(19) Europäisches Patentamt
European Patent Office
Office européen des brevets

(11) Veröffentlichungsnummer: **0 115 314**
**B1**

(12) # EUROPÄISCHE PATENTSCHRIFT

(45) Veröffentlichungstag der Patentschrift: **08.06.88**

(21) Anmeldenummer: **84100664.6**

(22) Anmeldetag: **23.01.84**

(51) Int. Cl.⁴: **C 07 C 172/00,** C 07 C 35/32, C 07 D 303/14, C 07 D 319/06, C 07 F 7/18

(54) **Verfahren zur Herstellung von Cholecalciferolderivaten.**

(30) Priorität: **28.01.83 US 461836**

(43) Veröffentlichungstag der Anmeldung: **08.08.84 Patentblatt 84/32**

(45) Bekanntmachung des Hinweises auf die Patenterteilung: **08.06.88 Patentblatt 88/23**

(84) Benannte Vertragsstaaten: **AT BE CH DE FR GB IT LI NL**

(56) Entgegenhaltungen:
**CHEMICAL ABSTRACTS, Band 87, Nr. 25, 20.12.1982, Seite 891, Zusammenfassung 21656j,Columbus, Ohio, US,J.J. PARTRIDGE et al.: "Synthesis and structure proof of 23S, 25-dihydroxycholecalciferol, a new in vivo vitamin D3 metabolite"**
**CHEMICAL ABSTRACTS, Band 97, Nr. 25, 20.12.1982, Seiten 891-892,Zusammenfassung 21562k,Columbus, Ohio, US,E.G. BAGGIOLINI et al.: "Preparation of 1 alpha-hydroxylated vitamin D metabolites by total synthesis"**
**TETRAHEDRON LETTERS, Nr. 41, Oktober 1977, Pergamon Press, Oxford, GB,BASIL LYTHGOE et al.: "Direct total synthesis of vitamins D2 and D3", Seiten 3685-3688**

**Die Akte enthält technische Angaben, die nach dem Eingang der Anmeldung eingereicht wurden und die nicht in dieser Patentschrift enthalten sind.**

(73) Patentinhaber: **F. HOFFMANN- LA ROCHE & CO. Aktiengesellschaft, CH- 4002 Basel (CH)**

(72) Erfinder: **Baggiolini, Enrico Giuseppe, 30 Evergreen Drive, North Caldwell, N.J. (US)**
Erfinder: **Uskokovic, Milan Radoje, 253 Highland Avenue, Upper Montclair, N.J. (US)**
Erfinder: **Wovkulich, Peter Michael, 124 Rhoda Avenue, Nutley, N.J. (US)**

(74) Vertreter: **Mahé, Jean, Postfach 3255 Grenzacherstrasse 124, CH- 4002 Basel (CH)**

**EP 0 115 314 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft das 1α,23R,25-Trihydroxycholecalciferol der Formel

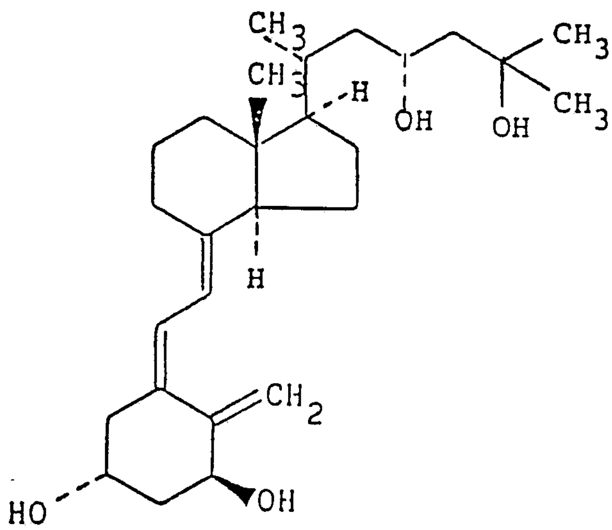

I

ein Verfahren zu dessen Hestellung, sowie neue in diesem Verfahren vorkommende Zwischenprodukte. In CA 97: 216561j sind keine 1α-hydroxylierte Verbindungen und in Tetrahedron Letters 1977, 3685-3688 keine 1α-, 23- oder 25-hydroxylierte Verbindungen beschrieben. Andererseits betrifft CA 97: 216562k keine Verbindungen mit einer freien 23-Hydroxygruppe. Die Verbindung der Formel I kann zur Behandlung von Krankheiten die durch einen Mangel an 1,25-Dihydroxycholecalciferol verursacht sind, verwendet werden. Das Verfahren der vorliegenden Erfindung ist dadurch gekennzeichnet, dass man

a) eine Verbindung der Formel

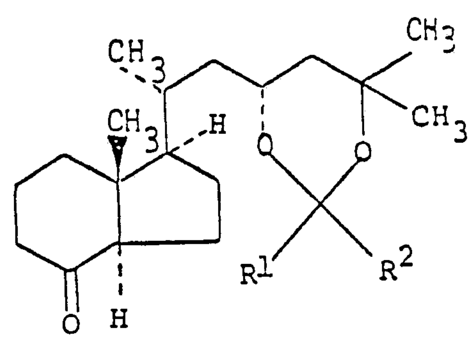

II

worin $R^1$ und $R^2$ Wasserstoff, Niederalkyl oder Aryl, oder zusammen $C_{3-6}$-Alkylen sind,

mit einer Verbindung der Formel

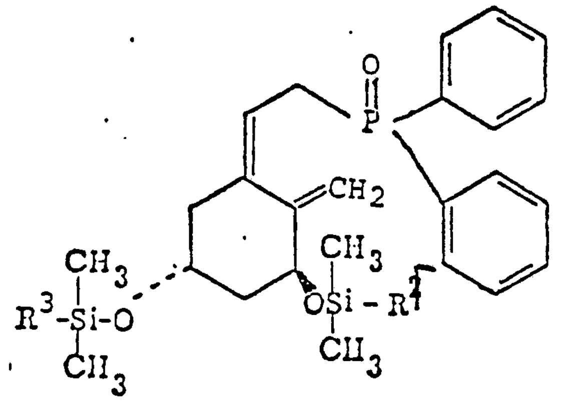

III

worin $R_3$ Niederalkyl oder Aryl ist, zu einer Verbindung - mit R-Konfiguration in 23-Stellung - der Formel

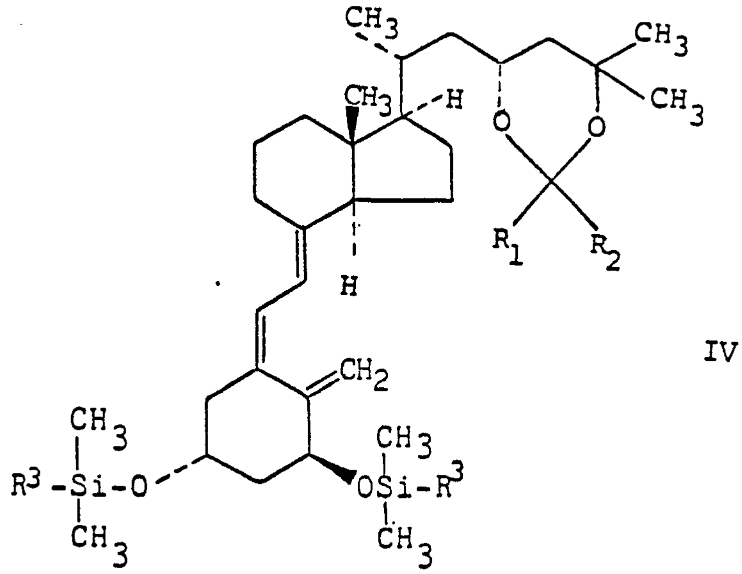

IV

worin $R^1$, $R^2$ und $R^3$ die obige Bedeutung haben, umsetzt und

b) die Schutzgruppen aus der Verbindung der Formel IV abspaltet.

Im Rahmen der vorliegenden Erfindung bezeichnet der Ausdruck "nieder" geradkettige oder verzweigte Gruppen mit bis zu 8 Kohlenstoffatome. Beispiele von Niederalkylgruppen sind Methyl, Äthyl, n-Propyl, i-Propyl, t-Butyl, Hexyl, Heptyl und Octyl. $C_{3-6}$-Alkylengruppen können geradkettig oder verzweigt sein. Beispiele davon sind Propylen, Butylen, Amylen und Hexylen.

Der Ausdruck "Aryl" bezeichnet eine aromatische Gruppe, wie Phenyl, die durch Niederalkyl, Fluor, Chlor, Brom, Jod, Nitro, Cyan oder Trifluormethyl mehrfach oder einfach substituiert sein kann.

Die Reaktionsstufe a) kann nach an sich bekannten Methoden, z. B. in Gegenwart einer Base, in einem Ätherlösungsmittel unter einer inerten Atmosphäre bei einer Temperatur im Bereich von etwa -80 bis -50°C durchgeführt werden. Beispiele von Basen sind Alkyllithiumverbindungen und Alkalimetalldialkylamide. Die Verbindung der Formel IV kann durch Elutionschromatographie auf Silicagel gereinigt werden.

Die Verfahrensstufe b) kann durch Behandlung der Verbindung der Formel IV mit einem Alkohol oder Wasser in Gegenwart einer Säure durchgeführt werden. Es kann dabei eine Mineralsäure, eine Niederalkancarbonsäure oder eine Sulfonsäure, vorzugsweise ein Kationenaustauscherharz in Form einer Suspension in einem niederen Alkanol verwendet werden. Das Produkt der Formel I kann durch Filtrieren des festen Kationenaustauscherharzes und Abdampfen von flüchtigen Produkten unter vermindertem Druck gereinigt werden.

Die Verbindungen der Formel II und deren 23S- Isomeren können nach dem folgenden Verfahren hergestellt werden.

Eine Äthylidenverbindung der Formel

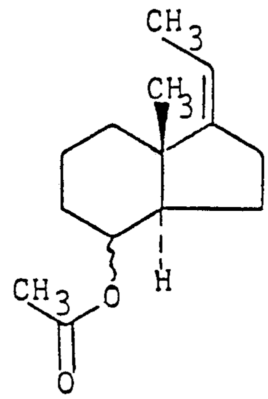

XII

wird in einer En-Reaktion mit einem Niederalkyl-2-haloacrylat umgesetzt. Man erhält stereoselektiv in Bezug auf die γ-Position der Buttersäure-niederalkylester-Seitenkette eine Verbindung der Formel

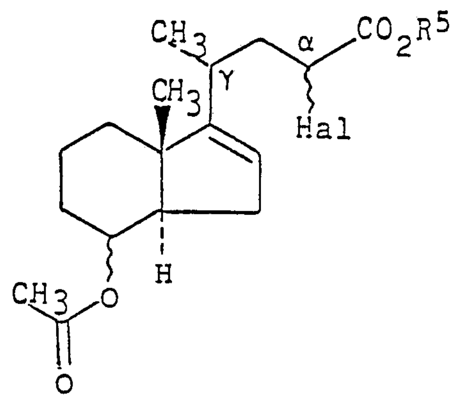

XIII

worin $R^5$ Niederalkyl ist.

Vorzugsweise wird diese Reaktion in Gegenwart einer Lewis-Säure in einem inerten Lösungsmittel bei einer Temperatur im Bereich von etwa -20 bis 100°C, vorzugsweise von etwa 0°C bis Raumtemperatur durchgeführt. Beispiele von inerten Lösungsmittel sind Methylenchlorid, Tetrachlorkohlenstoff, Chloroform, aromatische Kohlenwasserstoffe, wie Benzol oder Toluol, und niederaliphatische Kohlenwasserstoffe, wie Hexan, Heptan oder Octan. Als Lewis-Säuren kann man Niederalkylaluminiumdihalogenide und Aluminiumtrihalogenide in einer schwachen Base, z. B. Äthylaluminiumdichlorid, Aluminiumtribromid oder Aluminiumchlorid in Pyridin verwenden.

Äthylaluminiumdichlorid ist bevorzugt.

Das Gemisch der Stereoisomeren kann durch Elutionschromatographie aufgetrennt werden. Man erhält ein 1: 6-Gemisch von R- bzw. S-Stereoisomeren an der α-Position.

Ein R- oder S-Isomer oder ein Gemisch davon kann durch Äquilibrierung in ein 1 : 1-Gemisch der R- und S-Isomere übergeführt werden. Die Äquilibrierung wird durch Umsetzung mit einer Verbindung der Formel XIII mit Lithiumbromid durchgeführt. Die Temperatur der Reaktion ist nicht kritisch. Zweckmässig wird bei Raumtemperatur in einem polaren Lösungsmittel, wie Aceton, Dimethylformamid, Acetonitril, Dimethylsulfoxid oder Tetrahydrofuran, gearbeitet.

In der nächsten Stufe wird die Verbindung der Formel XIII durch Reaktion mit einem Hydrid in einem inerten organischen Lösungsmittel zu einer Verbindung der Formel

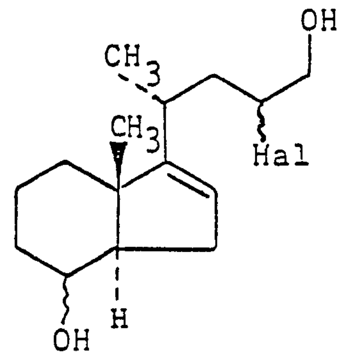

XIV

übergeführt.

Beispiele von Hydriden sind Lithiumaluminiumhydrid oder vorzugsweise Diisobutylaluminiumhydrid. Beispiele von inerten organischen Lösungsmitteln sind niederaliphatische Kohlenwasserstoffe, wie Hexan, Heptan oder Octan, herkömmliche Ätherlösungsmittel, wie Diäthyläther oder Tetrahydrofuran, und aromatische Kohlenwasserstoffe, wie Benzol oder Toluol, oder Niederalkylhalogenide, wie Methylenchlorid, Chloroform oder Tetrachlorkohlenstoff. Die Reaktion wird bei einer Temperatur im Bereich von etwa -70 bis 80° C vorzugsweise etwa 0° C bis Raumtemperatur, durchgeführt.

In der nächsten Stufe wird die Verbindung der Formel XIV mit einer Base in einem Lösungsmittel bei einer Temperatur im Bereich von etwa -70 bis 80° C, vorzugsweise etwa 0° C bis Raumtemperatur, zu einer Verbindung der Formel

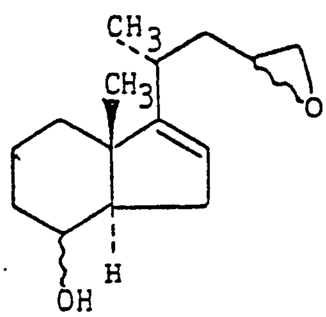

XV

umgesetzt.

Beispiele von Basen sind Kalium- oder Natrium-t-butoxid, Kaliumisopropoxid, Alkalimetallhydroxide, wie Natrium- oder Kaliumhydroxid und tri-Niederalkylamine. Beispiele von Lösungsmittel sind Niederalkanole, wie Methanol, Äthanol, t-Butanol oder Isopropanol, und herkömmliche Äther, wie Diäthyläther oder Tetrahydrofuran.

In der nächsten Stufe wird eine Verbindung der Formel XV mit Wasserstoff in Gegenwart eines Hydrierungskatalysators in einem inerten organischen Lösungsmittel, bei einer Temperatur im Bereich von etwa 0 bis 80° C, vorzugsweise bei Raumtemperatur, zu einer Verbindung der Formel

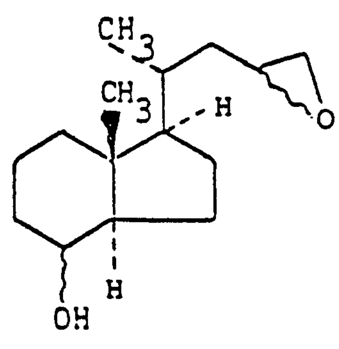

V

umgesetzt.

Beispiele von Hydrierungskatalysatoren sind 5 % Platin auf Kohlenstoff, 5-10 % Palladium auf Kohlenstoff, 5-10 % Rhodium auf Kohlenstoff oder Platinoxid. Beispiele von Lösungsmittel sind niederaliphatische Kohlenwasserstoffe, wie Hexan, Heptan oder Octan, Niederalkanole, wie Methanol, Äthanol oder Propanol, herkömmliche Äther, wie Diäthyläther oder Tetrahydrofuran, aromatische Kohlenwasserstoffe, wie Benzol oder

Toluol, und Alkancarbonsäure-niederalkylester, wie Äthylformiat oder Äthylacetat.

Gewünschtenfalls wird in der nächsten Stufe eine Verbindung der Formel V mit einem Silylierungsmittel unter einer inerten Atmosphäre bei einer Temperatur im Bereich von etwa 0 bis 80°C, vorzugsweise bei Raumtemperatur, zu einer Verbindung der Formel

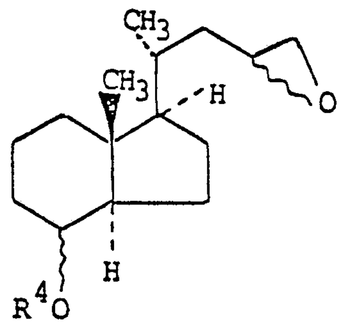

VI

worin $R^4$ tri-Niederalkylsilyl, di-Niederalkylarylsilyl, Niederalkyldiarylsilyl oder Triarylsilyl ist, umgesetzt. Beispiele von Silylierungsmittel sind Trimethylchlorsilan, Triäthylchlorsilan, t-Butyldimethylchlorsilan, Chlordimethylphenylsilan, Chlortriphenylsilan und, vorzugsweise Trimethylsilylimidazol.

In der nächsten Stufe wird die Verbindung der Formel V oder VI mit einem Propenylierungsmittel, wie Lithiumdiisopropenylcuprat, 2-Propenyllithium oder 2-Propenylmagnesium-bromid, zu einer Verbindung der Formel

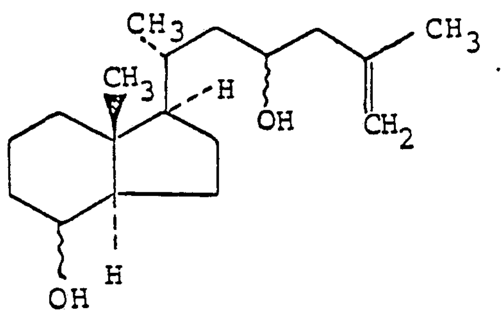

VII

oder

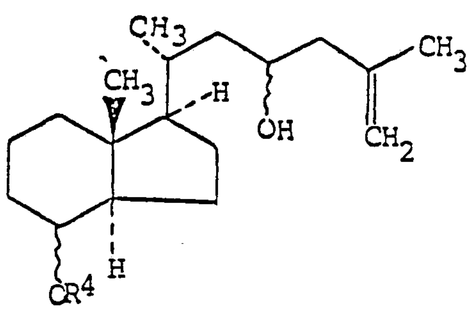

VIII

worin $R^4$ die obige Bedeutung hat, umgesetzt.

Die Reaktion wird in einem herkömmlichen Ätherlösungsmittel, wie Diäthyläther oder Tetrahydrofuran, oder in einem niederen Kohlenwasserstoff-Ätherlösungsmittel, bei einer Temperatur im Bereich von etwa -70°C bis Raumtemperatur, vorzugsweise von etwa -50 bis -5°C, durchgeführt.

Wird eine Verbindung der Formel VIII hergestellt, so wird in der nächsten Stufe die Gruppe $R^4$ durch Reaktion mit einem Fluoridionen enthaltenden Reagenz, wie Kaliumfluorid oder Tetra-n-butylammoniumfluorid, oder einer verdünnten Mineralsäure, wie Salzsäure, Schwefelsäure oder Perchlorsäure, in einem Lösungsmittel, wie einem wässrigen Äther oder nieder-Alkohol, abgespalten, wobei man eine Verbindung der Formel VII erhält.

Ein Gemisch der R* - und S* -Diastereomeren der Formel VII kann durch Elutionschromatographie auf Silicagel aufgetrennt werden.

In der nächsten Stufe wird eine Verbindung der Formel VII mit einem Epoxidierungsmittel bei einer

Temperatur im Bereich von etwa -70 bis 80° C, vorzugsweise von 0° C bis Raumtemperatur, zu einer Verbindung der Formel

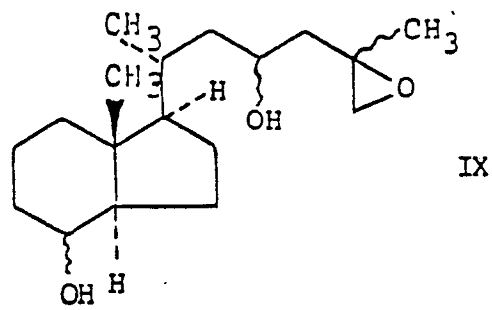

IX

umgesetzt.

Beispiele von Epoxidierungsmittel sind m-Chlorperbenzoesäure, Peressigsäure, t-Butylhydroperoxid, in Gegenwart eines Übergangsmetallkatalysatoren, wie Molybdenhexacarbonyl. Die m-Chlorperbenzoesäure ist bevorzugt.

In der nächsten Stufe wird die Verbindung der Formel IX durch Behandlung mit einem Reduktionsmittel, in eine Verbindung der Formel

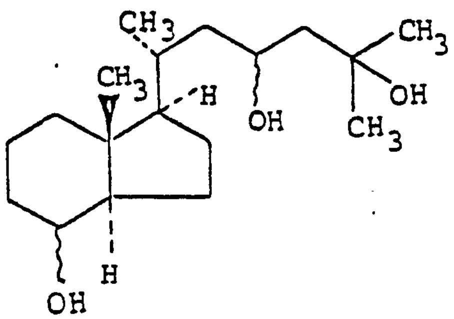

X

übergeführt. Bei dieser Reaktion können die für die Reduktion von Epoxiden zu Alkohole herkömmlichen Reduktionsmittel verwendet werden. Bevorzugte Reduktionsmittel sind Hydride, wie Lithiumaluminiumhydrid, Natriumborhydrid, Diisobutylaluminiumhydrid und Lithium-tri-t-butoxy-aluminiumhydrid.

In der nächsten Stufe wird die Verbindung der Formel X durch Behandlung mit einem für die Überführung eines 1,3-Diols in das entsprechende 1,3-Dioxan herkömmlichen Reagenz in die Verbindung der Formel

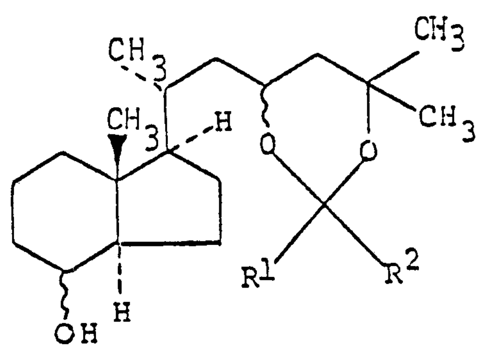

XI

worin $R^1$ und $R^2$ die obige Bedeutung haben, übergeführt.

Bevorzugte Reagenzien sind Ketone und Aldehyde und ein Dehydrierungsmittel oder ein Vinyläther in Gegenwart eines sauren Kata-lysators. Beispiele von sauren Katalysatoren sind Toluol, Sulfonsäuremonohydrat, Oxalsäure, Trifluoressigsäure, anorganische Säuren, z. B. Salzsäure, und kationische Ionenaustauscherharzen. Beispiele von Dehydrierungsmittel sind wasserfreies Kupfer(II)sulfat, wasserfreies Magnesiumsulfat und Molekularsieben. Beispiele von Vinyläther sind Methyl oder Äthylvinyläther, Methylcyclohexenyläther und Methyl-2-propenyläther. Beispiele von Ketonen sind Aceton und Cyclohexanon. Beispiele von Aldehyden sind Formaldehyd, Acetaldehyd und Benzaldehyd. Die Reaktion kann in einem inerten Lösungsmittel, wie Hexan, Heptan, Octan, Benzol, Toluol, Methylenchlorid, Äther oder Äthylacetat, durchgeführt werden.

In der nächsten Stufe wird die Verbindung der Formel XI durch Behandlung mit einem Oxidierungsmittel in eine Verbindung der Formel II bzw. das entsprechende 23(S)-Isomer übergeführt. Beispiele von Oxidierungsmittel sind Chromverbindungen, wie Pyridiniumchlorochromat, 2,3-Bipyridiniumchlorochromat oder Pyridinchromtrioxid, oder aktiviertes Dimethylsulfoxid.

Herstellung der αR- und αS- Isomeren einen Verbindung der Formel XIII.

Einer Lösung von 6,8 g (-)[3aR[(Z),3aα,4a,7aα]-1-Äthyliden-octahydro-7a-methyl-1H-4-indenolacetat (94,4 % reines Isomer), 200 ml Methylenchlorid und 58 ml einer 0,827M Lösung von Methyl-α-bromacrylat in Methylenchlorid wurden unter trockener Argonatmosphäre unter Abkühlen im Eisbad tropfenweise 51,9 ml einer 1,49M Lösung von Äthylaluminiumdichlorid in Hexan zugesetzt. Das Eisbad wurde entfernt und das Gemisch wurde bei Raumtemperatur gerührt, dann in 250 ml 10 %-ige wässrige Kaliumnatriumtartratlösung enthaltendes Eis geschüttet und dann mit 3 x 250 ml Methylenchlorid extrahiert. Die vereinigten Extrakte wurden filtriert, dann nacheinander mit Wasser und Salzlauge gewaschen und über Natriumsulfat getrocknet. Nach Filtrieren und Entfernen des Lösungsmittels im Vakuum erhielt man 16 g rohes Produkt. Durch Chromatographie auf 150 g Silicagel mit Hexan/Äthylacetat als Eluierungsmittel erhielt man 8,5 g eines 13 : 87-Gemisches von [3aS-[3aα,3(R*, R*),7β,7aβ]-7-(Acetyloxy)-α-brom-3a, 4, 5, 6, 7, 7a-hexahydro-γ,3a-dimethyl-1H-inden-3-butansäuremethylester und dessen αS-Isomer. Das Gemisch wurde mit 9,53 g (0,11 Mol) wasserfreiem Lithiumbromid in 300 ml Aceton bei Raumtemperatur gerührt. Das Lösungsmittel wurde im Vakuum entfernt. 200 ml Wasser wurden dem Rückstand zugesetzt und das Gemisch wurde mit 2 x 100 ml Methylenchlorid extrahiert. Die vereinigten Extrakte wurden nacheinander mit Wasser und Salzlauge gewaschen und über wasserfreiem Natriumsulfat getrocknet. Filtrieren gefolgt durch Entfernen des Lösungsmittels im Vakuum ergaben 8,2 g Produkt in Form eines 1 : 1-Gemisches von 3aS-[3aα,3(R*,R*),7β,7aβ]-7-(Acetyloxy)-α-brom-3a, 4, 5, 6, 7, 7a-hexahydro-γ,3a-dimethyl-1H-inden-3-butansäuremethylester und dessen αS-Isomer. Die Trennung der beiden Isomeren erfolgte durch Mitteldruckflüssigchromatographie von 1-2 g Portionen in einer mit Silicagel gefüllten Säule mit Hexan/Äthylacetat als Eluierungsmittel. Das polarere αS-Isomere wurde anschliessend noch 3 x der Lithiumbromid/Aceton-Äquilibrierung-Trennungsequenz unterworfen und man erhielt dabei insgesamt 7,1 g (63 %) 3aS-[3aα,3(R*,R*) 7β, 7aβ]-7-(Acetyloxy)-α-brom-3a, 4, 5, 6, 7, 7a-hexahydro-γ 3a-dimethyl-1H-inden-3-butansäuremethylester, Smp. 62°C nach Umkristallisation aus Hexan/Äther; $[\alpha]_D^{25}$ + 41,14° (c, 1,0063, $CHCl_3$).

In einem getrennten Experiment wurde die analytische Menge des anderen Isomers, 3aS-[3aα,3(R*,S*),7β,7aβ]-7-(Acetyloxy)-α-brom-3a, 4, 5, 6, 7, 7a-hexahydro-γ,3a-dimethyl-1H-inden-3-butansäuremethylester, in Form eines Öls, $[\alpha]_D^{25}$ -40,91° (c, 0,9925 $CHCl_3$) unter Ausschaltung der Lithiumbromid/Aceton-Äquilibrierungsstufe erhalten.

**Refenzbeispiel**

a) Einer Lösung von 92,2 ml 20 % Diisobutylaluminiumhydrid in Hexan und 90 ml Tetrahydrofuran wurde bei 0°C eine Lösung von 6,7 g 3aS-[3aα,3(R*,R*),7β,7aβ]-7-(Acetyloxy)-α-brom-3a, 4, 5, 6, 7, 7a-hexahydro-γ,3a-dimethyl-1H-inden-3-butansäure-methylester in 80 ml trockenem Tetrahydrofuran zugesetzt. Das Eisbad wurde entfernt und das Gemisch wurde auf Raumtemperatur wärmen gelassen. Das Gemisch wurde in 57 ml Eiswasser gegossen, mit 1N-Salzsäure angesäuert und dann mit 3 x 250 ml Äthylacetat extrahiert. Die Extrakte wurden mit Wasser und Salzlauge bis zur Neutralität gewaschen und dann über wasserfreiem Natriumsulfat getrocknet. Nach Filtrieren und Abdampfen des Lösungsmittels wurde der Rückstand auf Silicagel mit Hexan/Äthylacetat als Eluierungsmittel chromatographiert. Man erhielt 5 g (91 %) [3aS-[3aα, 3(R*,R*),7β,7aβ]]-3-(3-Brom-4-hydroxy-1-methylbutyl)hexahydro-3a-methyl-1H-inden-7-ol in Form eines Öls $[\alpha]_D^{25}$ + 19,88° (c, 0,5532, $CHCl_3$).

b) Einer Lösung von 4,6 g [3aS-[3aα,3(R*,R*),7β,7aβ]]-3-(3-Brom-4-hydroxy-1-methylbutyl)hexahydro-3a-methyl-1H-inden-7-ol in 145 ml trockenem t-Butanol wurden unter Argonatmosphäre bei Raumtemperatur 2,1 g Kalium-t-butoxid zugesetzt. Nach Rühren wurde das Gemisch im Eisbad abgekühlt und 10 ml gesättigtes wässriges Natriumdihydrogenphosphat wurden zugesetzt. Das Gemisch wurde gerührt, dann in 500 ml Eiswasser gegossen und mit 3 x 200 ml Methylenchlorid extrahiert. Die vereinigten Extrakte wurden nacheinander mit Wasser und Salzlauge gewaschen, dann über wasserfreiem Natriumsulfat getrocknet. Nach Filtrieren und Abdampfen des Lösungsmittels im Vakuum erhielt man 3,5 g rohes Produkt. Durch Mitteldruckflüssigchromatographie mit Hexan/Äthylacetat als Eluierungsmittel erhielt man 2,9 g (84 %) [3aS[3aα, 3(R*,S*),7β,7aβ]]-Hexahydro-3a-methyl-3-(1-methyl-2-oxiranyl-äthyl)-1H-inden-7-ol, Smp. 62,5-63°C nach Umkristallisation aus Hexan/Äthylacetat; $[\alpha]_D^{25}$ -31,26° (c, 1,03, $CHCl_3$).

3) Ein Gemisch von 2,9 g [3aS-[3aα,3(R*,S*),7β,7aβ]]-Hexahydro-3a-methyl-3-(1-methyl-2-oxiranyläthyl)-1H-inden-7-ol, 0,29 g 5 % Platin auf Kohlenstoffkatalysator und 125 ml Äthylacetat wurde bei Raumtemperatur unter einer Wasserstoffatmosphäre gerührt. Nach Ende der Wasserstoffaufnahme wurde das Gemisch filtriert und das Lösungsmittel im Vakuum entfernt. Der Rückstand wurde auf Silicagel mit Hexan/Äthylacetat als Eluierungsmittel chromatographiert. Man erhielt 2,1 g (72 %) [1R-[1α(R*,S*),3aβ,4β,7aα]-Octahydro-7a-methyl-1-(1-methyl-2-oxiranyläthyl)-1H-inden-4-ol in Form eines Öls, $[\alpha]_D^{25}$ -3,18° (c, 1,0992, $CHCl_3$).

d) Einer Lösung von 1,4 g [1R-[1α(R*,S*),3aβ,4β,7aα]]-Octahydro-7a-methyl-1-(1-methyl-2-oxiranyläthyl)-1H-inden-4-ol und 40 ml Äthylacetat wurden 1,64 g Trimethylsilylimidazol zugesetzt. Das Gemisch wurde unter einer trockenen Argonatmosphäre bei Raumtemperatur gerührt, dann in 100 ml kaltem Wasser geschüttet und mit 3 x 25 ml Heptan/Äthylacetat extrahiert. Die Extrakte wurden mit 3 x 50 ml Wasser gewaschen und über wasserfreiem Natriumsulfat getrocknet. Das Gemisch wurde filtriert und das Lösungsmittel im Vakuum entfernt. Chromatographie auf Silicagel mit Hexan/Äthylacetat als Eluierungsmittel ergab 1,81 g (99 %) [1R-[1α(R*, S*) 3aβ,4β,7aα]]-Octahydro-7a-methyl-1-(1-methyl-2-oxiranyl-äthyl)-1H-inden-4-ol-trimethylsilyläther. Dieses Produkt, welches Spuren von Ausgangsmaterial enthielt, und sich langsam zum Ausgangsmaterial umwandelte, wurde ohne weitere Reinigung in der nächsten Stufe verwendet.

e) Einer Lösung von 1,1 g 2-Brompropen und 15 ml wasserfreiem Diäthyläther wurden unter einer Argonatmosphäre bei -78°C 5,65 ml 1,6M t-Butyllithium in Pentan zugesetzt. Nach Rühren wurde dem Gemisch eine klare Lösung von Tributylphosphin/Kupfer(I) jodid in Äther tropfenweise zugesetzt. Nach Rühren wurden 0,292 g [1R-[1α(R*,S*),3aβ,4β, 7aα]]-Octahydro-7a-methyl-1-(1-methyl-2-oxiranyläthyl)-1H-inden-4-ol-trimethylsilyläther in 15 ml wasserfreiem Diäthyläther tropfenweise der bräunlichgelben Lösung zugesetzt. Das Gemisch wurde gerührt, dann das Abkühlungsbad durch ein Methanol/Wasser/Eisbad (-15 bis -10°C) ersetzt. Nach Rühren bei dieser Temperatur wurden 15 ml einer gesättigten wässrigen Natriumdihydrogenphosphatlösung zugesetzt. Nach Rühren wurde das Gemisch in 50 ml Wasser gegossen und mit 3 x 30 ml Diäthyläther extrahiert. Die vereinigten Extrakte wurden nacheinander mit Wasser und Salzlauge gewaschen und über wasserfreiem Natriumsulfat getrocknet. Filtrieren und Abdampfen des Lösungsmittels ergaben rohes Produkt in Form eines Öls. Dieses wurde zuerst auf 75 g Silicagel mit Hexan/äthylacetat als Eluierungsmittel chromatographiert. Man erhielt 0,253 g (77 %) rohes [1R-[1α(R*,S*),3aβ,4β,7aα]]-Octahydro-1-(3-hydroxy-1,5-dimethyl-5-hexenyl)-7a-methyl-1H-inden-4-ol-monotrimethyl-silyläther. Ohne weitere Reinigung wurde dieser mit 10 ml Tetrahydrofuran, 4 ml Methanol, 1 ml Wasser und 0,3 g Tetra-n-butylammoniumfluorid solange gerührt, bis die Reaktion als beendet angesehen wurde. Ungefähr die Hälfte des Lösungsmittels wurde im Vakuum entfernt. Das Gemisch wurde in 30 ml Wasser geschüttet, mit 3 x 20 ml Diäthyläther/ Hexan extrahiert und über wasserfreiem Natriumsulfat getrocknet. Filtrieren und Abdampfen des Lösungsmittels im Vakuum ergaben 0,191 g Produkt. Dieses wurde mit ausgehend von 0,20 g Ausgangsmaterial in einem ähnlichem Experiment erhaltenen Produkt kombiniert und auf Silicagel mit Hexan/ Äthylacetat als Eluierungsmittel chromatographiert. Man erhielt insgesamt 0,320 g (72 %) [1R-[1α(R*,S*),3aβ,4β,7aα]]-Octahydro-1-(3-hydroxy-1,5-dimethyl-5-hexenyl)-7a-methyl-1H-inden-4-ol, Smp. 98-88,5°C nach Umkristallisation aus Äthylacetat/Hexan, $[\alpha]_D^{25}$ +19,31° (c, 0,994, $CHCl_3$).

f) Einer Lösung von 0,9 g [1R-[1α(R*,S*)3aβ,4β,7aα]]-Octahydro-1-(3-hydroxy-1,5-dimethyl-5-hexenyl)-7a-methyl-1H-inden-4-ol in 10 ml Methylenchlorid wurden bei 0°C 0,09 g 85 % m-Chlorbenzoesäure zugesetzt. Das Abkühlungsbad wurde entfernt, das Gemisch wurde gerührt und dann wurden 0,045 g 85 % m-Chlorperbenzoesäure zugesetzt. Das Gemisch wurde in 50 ml Wasser geschüttet und mit 50 ml Methylenchlorid extrahiert. Der Extrakt wurde mit 2 x 25 ml 10 %-igem wässrigem Natriumsulfid, 2 x 25 ml eiskaltem 1N-Kaliumcarbonat und Wasser bis zur Neutralität, dann mit Salzlauge gewaschen und über wasserfreiem Natriumsulfat getrocknet. Filtrieren und Abdampfen des Lösungsmittels ergaben 1R-[1α(R*,S*,R*),3aβ,4β,7aα]-Octahydro-1-[3-hydroxy-1-methyl-4-(1-methyloxiranyl)butyl]-7a-methyl-1H-inden-4-ol und 1R-[1α-(R*,S*,S*),3aβ,4β,7aα]-Octahydro-1-[3-hydroxy-1-methyl-4-(1-methyloxiranyl)butyl]-7a-methyl-1H-inden-4-ol in Form eines 1 : 1-Gemisches. Dieses Produkt wurde ohne weitere Reinigung in der nächsten Stufe verwendet.

g) Einem Gemisch von 0,01 g Lithiumaluminiumhydrid und 10 ml trockenes Tetrahydrofuran wurde bei 0°C eine Lösung von 0,08 g der Epoxide von Beispiel 7 in 5 ml trockenem Tetrahydrofuran zugesetzt. Nach Rühren unter einer Argonatmosphäre wurde dem Gemisch 15 ml Äthylacetat gefolgt durch 5 ml gesättigter wässriger Natriumsulfatlösung zugesetzt. Nach Rühren wurde das Gemisch durch Zusatz von 0,5N-Salzsäure angesäuert, dann mit 4 x 25 ml Äthylacetat extrahiert. Die vereinigten Extrakte wurden mit Wasser bis zur Neutralität gefolgt von Salzlauge gewaschen und über Natriumsulfat getrocknet. Nach Filtrieren und Entfernen des Lösungsmittels erhielt man 0,081 g [1R-[1α(R*,S*),3aβ,4β,7aα]]-Octahydro-4-hydroxy-7a-methyl-1H-inden-1-yl)-1,6-dimethyl- 2,4-hexandiol erhalten. Das Produkt war von genügender Reinheit um in der nächsten Stufe ohne weitere Reinigung verwendet zu werden. Durch Kristallisation aus Hexan/Äthylacetat wurde eine analytische Probe erhalten, Smp. 140-141°C; $[\alpha]_D^{25}$ +25,44° (c, 0,2988, Dioxan).

h) Ein Gemisch von 0,45 g [1R-[1α(R*,S*),3aβ,4β,7aα]]-Octahydro-4-hydroxy-7a-methyl-1H-inden-1-yl)-1,6-dimethyl-2,4-hexandiol, 30 ml 2,2-Dimethoxypropan und 0,05 g p-Toluolsulfonsäuremonohydrat wurde unter einer Argonatmosphäre bei Raumtemperatur gerührt, dann wurden 3 ml einer gesättigten wässrigen Natriumbicarbonatlösung zugesetzt. Das Lösungsmittel wurde im Vakuum entfernt. 25 ml Wasser wurden dem Rückstand zugesetzt. Nach Extraktion mit 2 x 25 ml Äthylacetat wurden die vereinigten Extrakte nacheinander mit Wasser und Salzlauge gewaschen und über Natriumsulfat getrocknet. Filtrieren und Abdampfen des Lösungsmittel ergaben rohes Produkt in Form eines bernsteinfarbigen Öls. Dieses wurde auf Silicagel mit Hexan/Äthylacetat/Methylenchlorid als Eluierungsmittel chromatographiert. Man erhielt 0,36 g (71 %) IR-[1α(R*,S*),3aβ,4β,7aα]-Octahydro-1-[1-methyl-2-(2,2,4,4-tetramethyl-1,3-dioxan-6-yl)äthyl]-7a-methyl-1H-inden-4-ol in Form eines Feststoffs, Smp. 78-80°.

i) Eine Lösung von 0,101 g IR-[1α(R*,S*),3aβ,4β,7aα]-Octahydro-1-[1-methyl-2-(2,2,4,4-tetramethyl-1,3-dioxan-6-yl)äthyl]-7a-methyl-1H-inden-4-ol in 6 ml trockenem Methylenchlorid wurde mit 0,17 g wasserfreiem Natriumacetat und 0,345 g 2,2'-Bipyridiniumchlorchromat versetzt. Das Gemisch wurde bei Raumtemperatur gerührt, dann wurden 0,15 g 2,2'-Bipyridiniumchlorchromat zugesetzt und das Rühren wurde fortgesetzt. Nach Zugabe von Wasser wurde das Reaktionsgemisch mit 3 x 50 ml Äther extrahiert. Die vereinigten organischen

Phasen wurden mit Wasser gewaschen, getrocknet und eingedampft. Der rohe Rückstand wurde durch Chromatographie auf Silica mit Hexan/Äthylacetat als Eluierungsmittel chromatographiert. Man erhielt 0,09 g (90 %) reines 1R-[1α(R*,S*),3aβ,4β,7aα]-Octahydro-1-[1-methyl-2-(2,2,4,4-tetramethyl-1,3-dioxan-6-yl)äthyl]-7a-methyl-1H-inden-4-on.

j) Eine Lösung von 0,28 g [3S,3α,5β,Z)]-2-[2-Methylen-3,5-bis-[1,1-dimethyläthyl)dimethylsilyloxy]cyclohexyliden]äthyldiphenylphosphinoxid in 6 ml trockenem Tetrahydrofuran wurde bei -78°C unter einer Argonatmosphäre tropfenweise mit 0,275 ml einer 1,7M-Lösung von n-Butyllithium in Hexan versetzt. Nach Rühren wurde eine Lösung von 0,091 g IR-[1α-(R*,S*),3aβ,4β,7aα]-Octahydro-1-[1-methyl-2-(2,2,4,4-tetra-methyl-1,3-dioxan-6-yl)äthyl]-7a-methyl-1H-inden-4-on in 1,5 ml Tetrahydrofuran langsam zugesetzt und das Gemisch wurde bei -78°C gerührt. Dann wurde es mit 2 ml eines 1 : 1-Gemisches von 1N-Natriumbicarbonat und 1N-Kaliumnatrium-tartrat behandelt, auf Raumtemperatur abkühlen gelassen, mit Wasser verdünnt und mit 3 x 50 ml Äthylacetat extrahiert. Die vereinigten organischen Extrakte wurden mit Salzlauge gewaschen, getrocknet und eingedampft. Der Rückstand wurde durch Chromatographie auf Kieselerde mit Hexan/Äthylacetat als Eluierungsmittel gereinigt. Man erhielt 0,172 g (1α,3β,5Z,7E,23S)-8,10-Secocholesta-5,7,10(9)-trien-1,3-bis[(1,1-dimethyläthyl)-dimethylsilyloxy]-23,25-diol-(1-methyläthyliden)-acetal in Form eines trüben farblosen Öls, das direkt in der nächsten Stufe verwendet wurde.

k) Das Produkt von j) wurde in 9 ml trockenem Methanol gelöst, mit 3 g Kationenaustauschharz behandelt und gerührt. Nach Filtrieren und Waschen des Harzes mit 20 ml Methanol wurde das Lösungsmittel abgedampft und der Rückstand wurde in 80 ml Äthylacetat gelöst und mit 2 x 20 ml 2N-Natriumbicarbonatlösung, dann mit 3 x 20 ml Salzlauge gewaschen. Der erhaltene Rückstand wurde nach Abdampfen des Lösungsmittels durch Chromatographie auf Kieselerde mit Äthylacetat als Eluierungsmittel gereinigt. Man erhielt 0,106 g (90 %) reines 1α,23S,25-Trihydroxycholecalciferol in Form eines weissen, amorphen Pulvers, $[\alpha]_D^{25}$ +47,3° (c, 0,2, EtOH).

**Beispiel**

Nach dem Verfahren des Referenzbeispiels wurde [3aS-[3aα,3(R*,S*) 7β,7aβ]]-7-(Acetyloxy)-α-brom-3a 4, 5, 6, 7, 7a-hexahydro-γ,3a-dimethyl-1H-inden-3-buttersäuremethylester nacheinander in das

a) [3aS-[3aα,3(R*,S*),7β,7aβ]]-3-(3-Bromo-4-hydroxy-1-methylbutyl)-hexahydro-3a-methyl-1H-inden-7-ol, Smp. 153-154°C, $[\alpha]_D^{25}$ -69,13° (c, 0,3866, $CHCl_3$);

b) [3aS-[3aα,3(R*,R*),7β,7aβ]]-Hexahydro-3a-methyl-3-(1-methyl-2-oxiranylethyl)-1H-inden-7-ol, in Form eines Öls vom Siedepunkt 75°C, 0,075 mmHg; $[\alpha]_D^{25}$ - 16,39° (c, 0,3966 $CHCl_3$);

c) [1R-[1α(R*,R*),3aβ,4β,7aα]]-Octahydro-7a-methyl-1-(1-methyl-2-oxiranylethyl)-1H-inden-4-ol, in Form eines Öls, Siedepunkt 75°C, 0,1 mmHg; $[\alpha]_D^{25}$ + 11,11 (c, 0,4952, $CHCl_3$);

d) [IR-[1α(R*,R*),3aβ,4β,7aα]]-Octahydro-7a-methyl-1-(1-methyl-2-oxiranylethyl)-1H-inden-4-ol-trimethylsilyläther, in Form eines Öls, Siedepunkt 80°C, 0, 1 mmHg; $[\alpha]_D^{25}$ +14,24° (c, 0,878, $CHCl_3$);

e) [1R-[1α(R*,R*),3aβ,4β,7aα]]-Octahydro-1-(3-hydroxy-1,5-dimethyl-5-hexenyl)-7a-methyl-1H-inden-4-ol;

f) ein Gemisch von [1R-[1α(R*,R*,R*),3aβ,4β,7aα]]-Octa-hydro-1-[3-hydroxy-1-methyl-4-(1-methyloxiranyl)butyl]-7a-methyl-1H-inden-4-ol und [1R-[1α(R*,R*,S*),3aβ,4β,7aα]]-Octahydro-1-[3-hydroxy-1-methyl-4-(1-methyloxiranyl)butyl]-7a-methyl-1H-inden-4-ol;

g) [1R-[1α(R*, R*),3aβ,4β,7aα]]-Octahydro-4-hydroxy-7a-methyl-1H-inden-1-yl)-1,6-dimethyl-2,4-hexandiol;

h) [1R-[1α(R*,R*),3aβ,4β,7aα]]-Octahydro-1-[1-methyl-2 (2,2,4,4-tetramethyl-1,3-dioxan-6-yl)äthyl]-7a-methyl-1H-inden-4-ol;

i) [1R-[1α(R*,R*),3aβ,4β,7aα]]-Octahydro-1-[1-methyl-2-(2,2,4,4-tetramethyl-1,3-dioxan-6-yl)äthyl]- 7a-methyl-1H-inden-4-on;

j) (1α,3β,5Z,7E,23R)-9,10-Secocholesta-5,7,10(9)-trien-1,3-bis[(1,1-dimethyläthyl)-dimethylsilyloxy]-23,25-diol-(1-methyläthyliden)-acetal; und

k) (1α,3β,5Z,7E,23R)-9,10-Secocholesta-5,7 10(8)-trien-1, 3, 23, 25-tetrol, Synonym von 1α,23R,25-Trihydroxycholecalciferol übergeführt.

**Patentansprüche** für die Vertragsstaaten

BE, CH, DE, FR, GB, IT, LI, NL

1. Verfahren zur Herstellung des 1α,23 (R),25-Trihydroxy-cholecalciferols der Formel

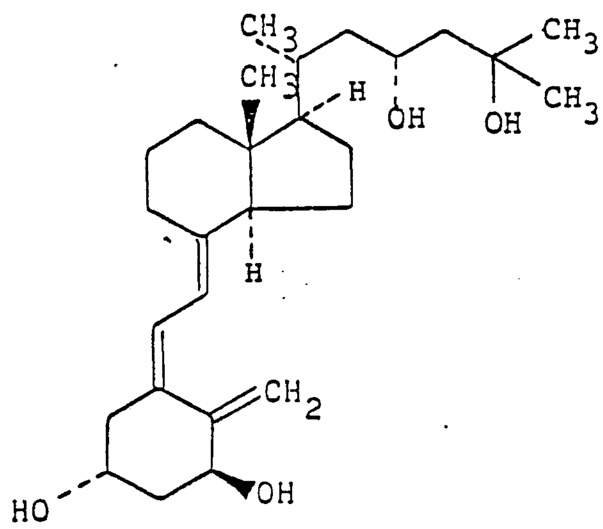

I

dadurch gekennzeichnet, dass man
a) eine Verbindung der Formel

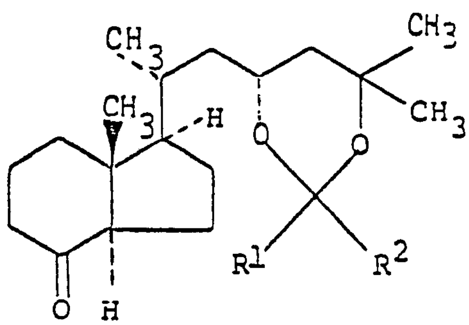

II

worin $R^1$ und $R^2$ Wasserstoff, Niederalkyl oder Aryl oder zusammen $C_{3-6}$ -Alkylen sind, mit einer Verbindung der Formel

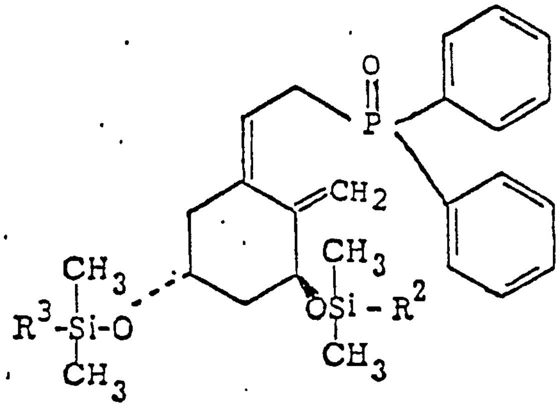

III

worin $R^3$ Niederalkyl oder Aryl ist, zu einer Verbindung der Formel

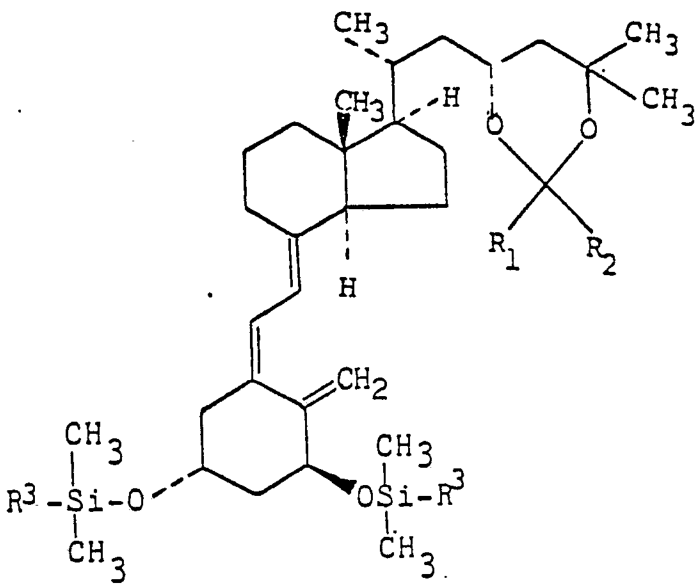

IV

worin $R^1$, $R^2$ und $R^3$ die obige Bedeutung haben, umsetzt und
b) die Schutzgruppen aus der Verbindung der Formel IV abspaltet.

2. Eine Verbindung der Formel

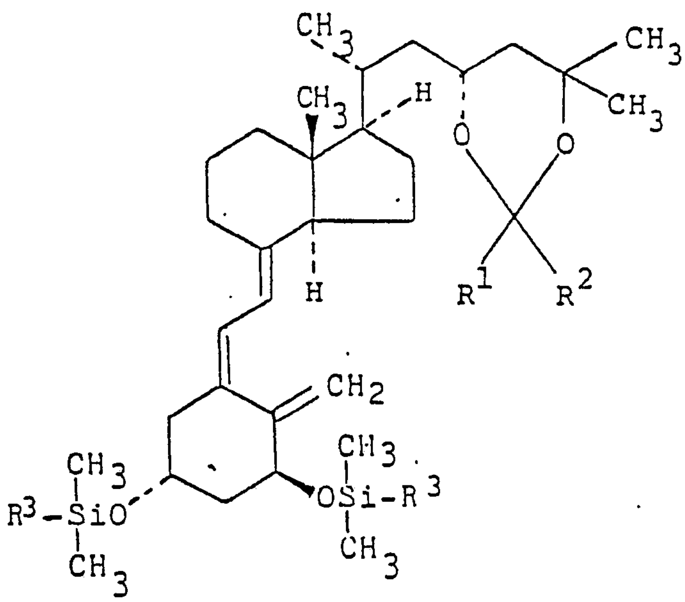

IV

worin $R^1$ und $R^2$ Niederalkyl, Aryl oder Wasserstoff sind mit R- Konfiguration in 23 - Stellung.

3. (1α,3β,5Z,7E,23R)-9,10-Secocholesta-5, 7, 10 (9)-trien-1,3-bis[(1,1-dimethyläthyl)-dimethylsilyloxy]-23,25-diol-(1-methyläthyliden)-cyclisches Acetal.

4. Das 1α,23(R),25-Trihydroxycholecalciferol.

**Patentanspruch** für den Vertragsstaat AT

Verfahren zur Herstellung des 1α,23 (R), 25-Trihydroxy-cholecalciferols der Formel

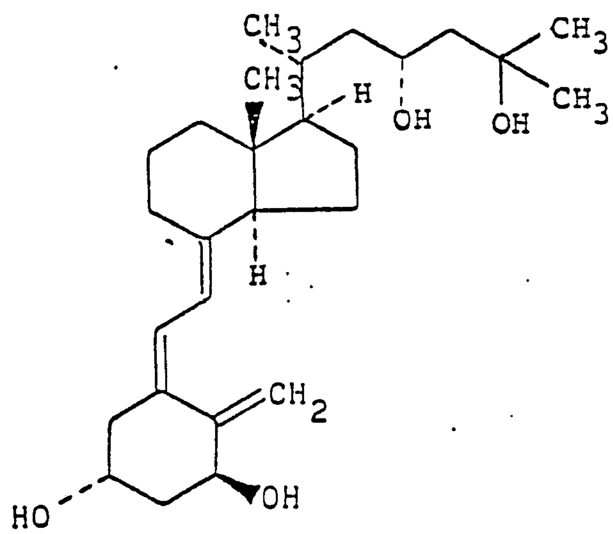

I

dadurch gekennzeichnet, dass man
a) eine Verbindung der Formel

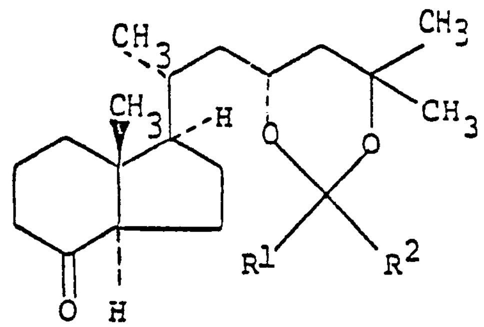

II

worin $R^1$ und $R^2$ Wasserstoff, Niederalkyl oder Aryl, oder zusammen $C_{3-6}$ -Alkylen sind, mit einer Verbindung der Formel

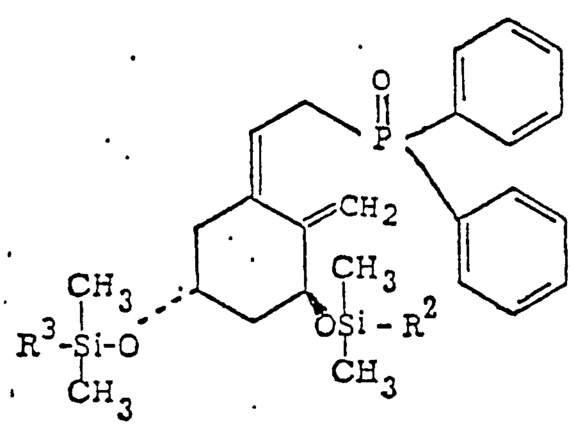

III

worin $R^3$ Niederalkyl oder Aryl ist, zu einer Verbindung der Formel

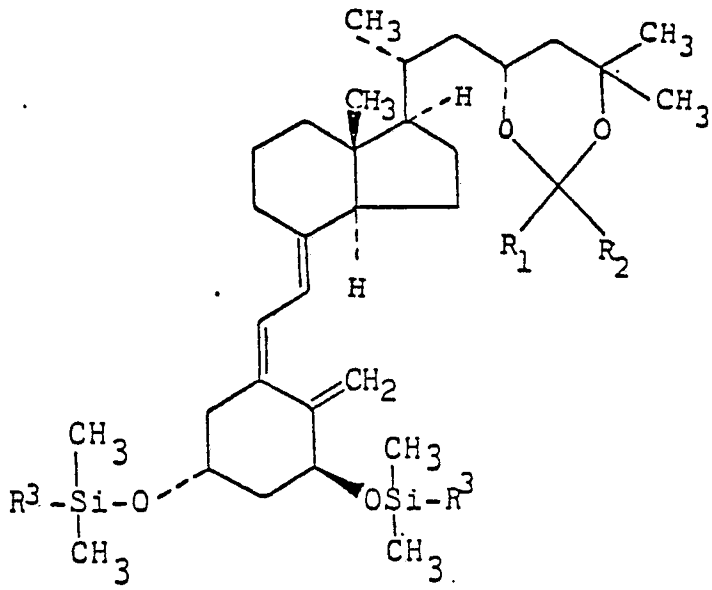

IV

worin $R^1$, $R^2$ und $R^3$ die obige Bedeutung haben, umsetzt und
b) die Schutzgruppen aus der Verbindung der Formel IV abspaltet.

**Claims** for the Contracting States BE, CH, DE, FR, GB, IT, LI, NL

1. A process for the manufacture of 1α,23(R),25-trihydroxycholecalciferol of the formula

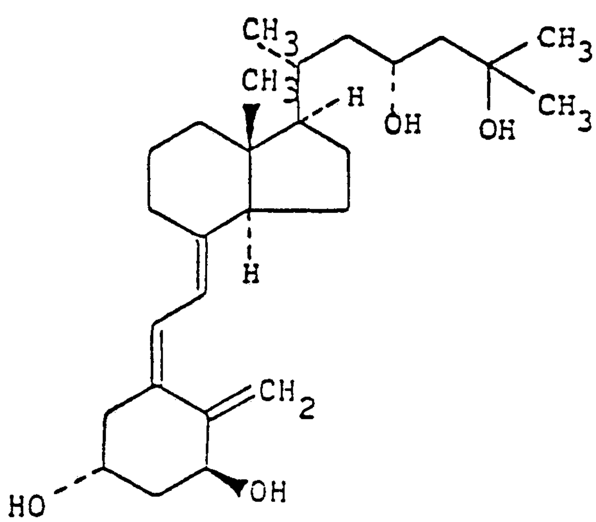

I

characterized by
a) reacting a compound of the formula

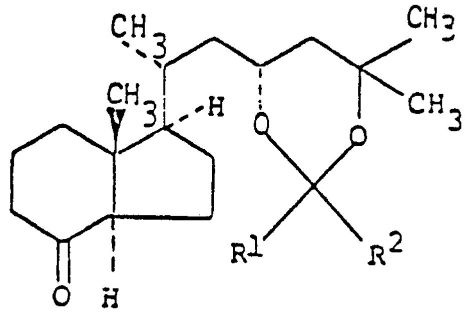

II

wherein $R^1$ and $R^2$ are hydrogen, lower alkyl or aryl or together are $C_{3-6}$-alkylene, with a compound of the formula

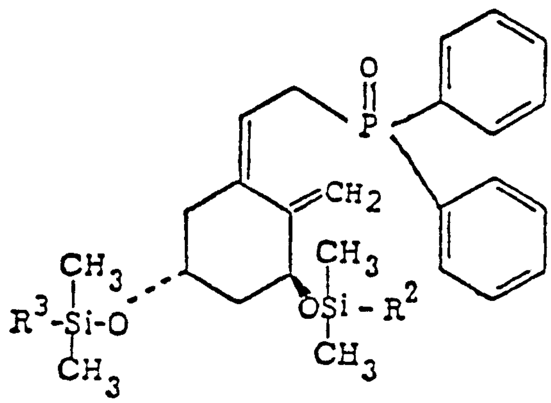

III

wherein $R^3$ is lower alkyl or aryl, to give a compound of the formula

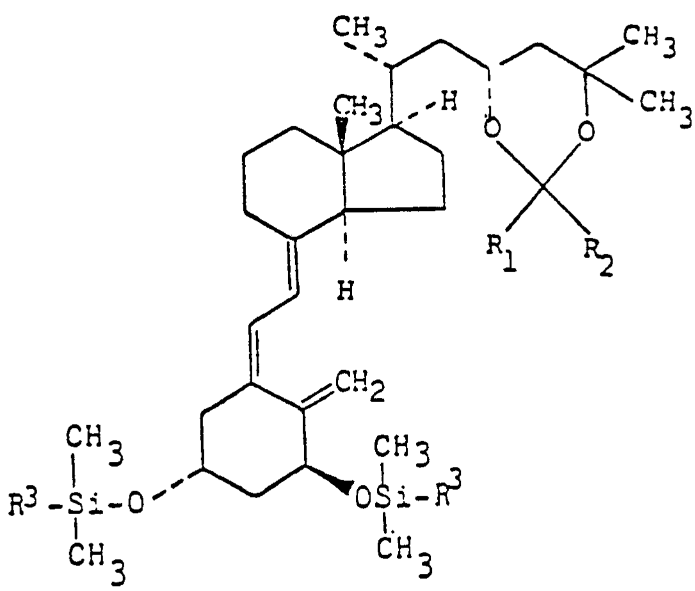

IV

wherein $R^1$, $R^2$ and $R^3$ have the above significance,
and
b) cleaving off the protecting groups from the compound of formula IV.
2. A compound of the formula

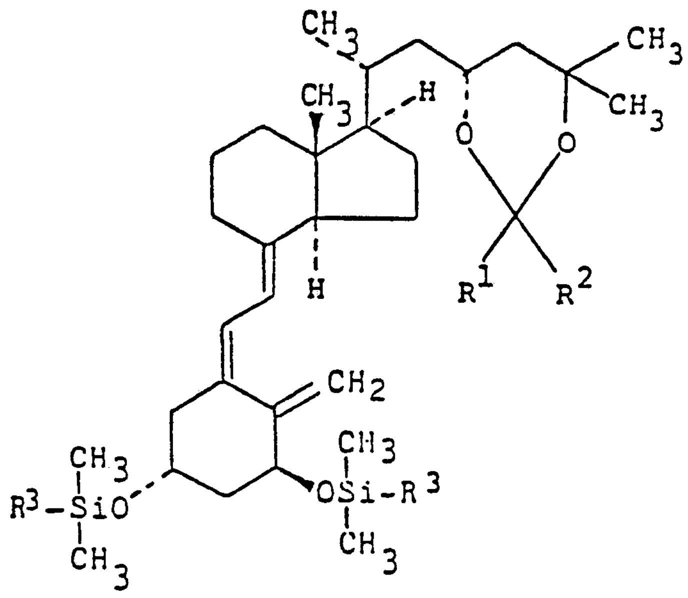

IV

wherein $R^1$ and $R^2$ are lower alkyl, arkyl or hydrogen, having the R-configuration in the 23-position.

3. (1α,3β,5Z,7E,23R)-9,10-Secocholesta-5,7,10(9)-triene-1,3-bis[(1,1-dimethylethyl)-dimethylsilyloxy]-23,25-diol (1-methylethylidene)-cyclic acetal.

4. 1α,23(R),25-Trihydroxycholecalciferol.

**Claim** for the contracting state AT

A process for the manufacture of 1α,23(R),25-trihydroxycholecalciferol of the formula

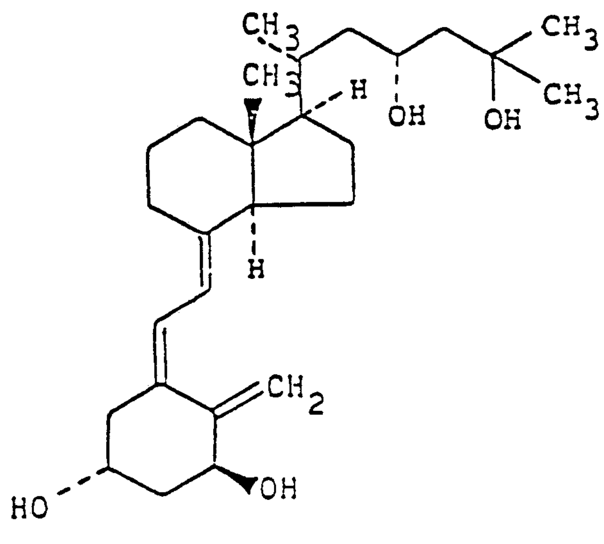

I

characterized by

a) reacting a compound of the formula

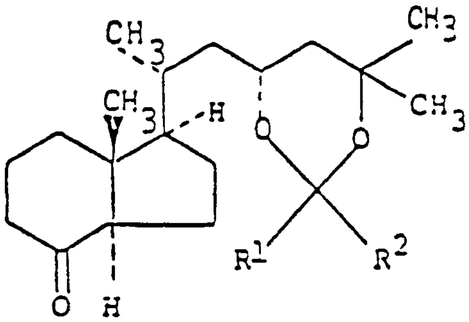

II

wherein $R^1$ and $R^2$ are hydrogen, lower alkyl or aryl or together are $C_{3-6}$-alkylene, with a compound of the formula

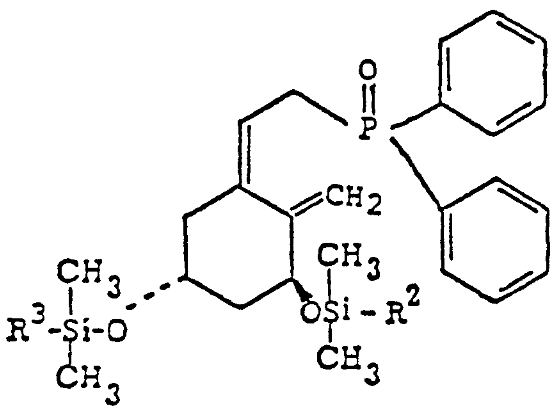

III

wherein $R^3$ is lower alkyl or aryl, to give a compound of the formula

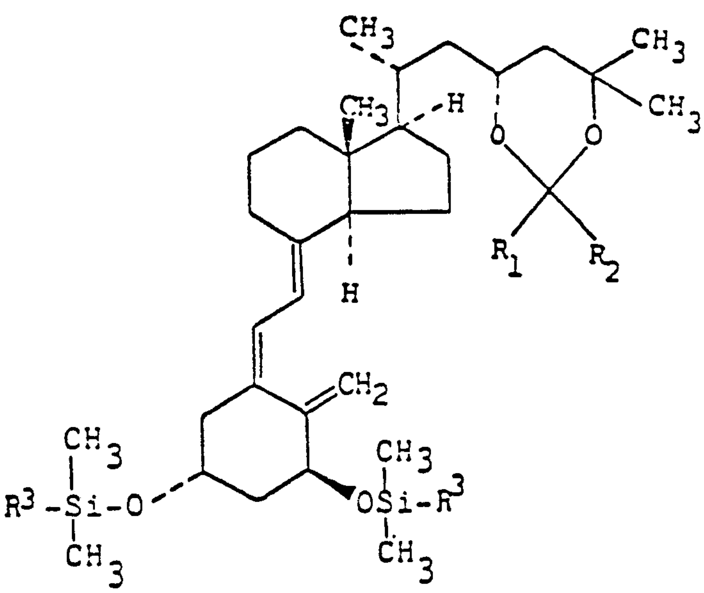

IV

wherein $R^1$, $R^2$ and $R^3$ have the above significance,
and
b) cleaving off the protecting groups from the compound of formula IV.

**Revendications** pour les contractants BE CH DE FR GB IT LI NL

1. Procédé de préparation du 1α,23(R),25-trihydroxy-cholécalciférol de formule

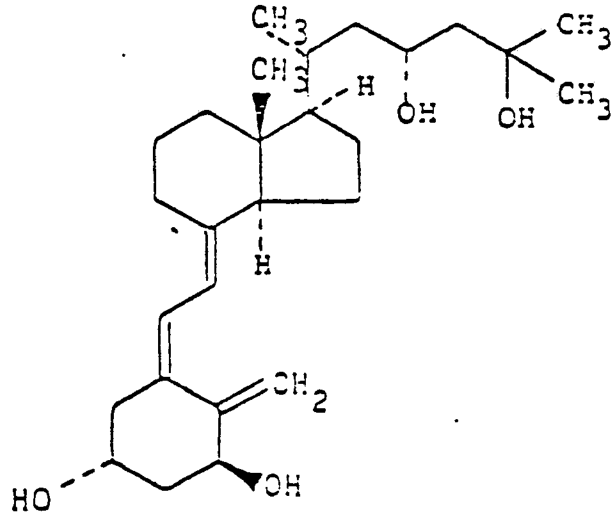

I

caractérisé en ce que
a) on fait réagir un composé de formula

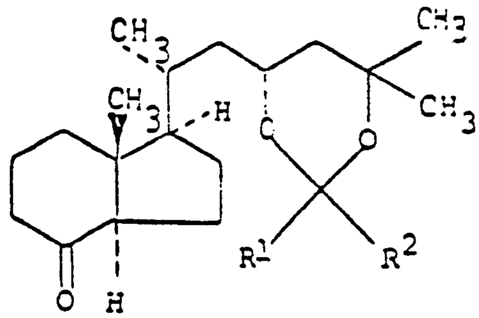

II

où R1 et R2 représentent ensemble un hydrogène, alcoyle inférieur ou aryle, ou ensemble un alcoylène en $C_3$ à $C_6$, avec un composé de formule

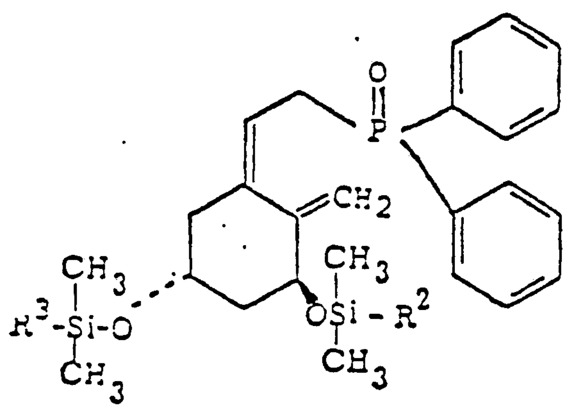

III

où R3 représente un alcoyle inférieur ou un aryle, pour donner un composé de formule

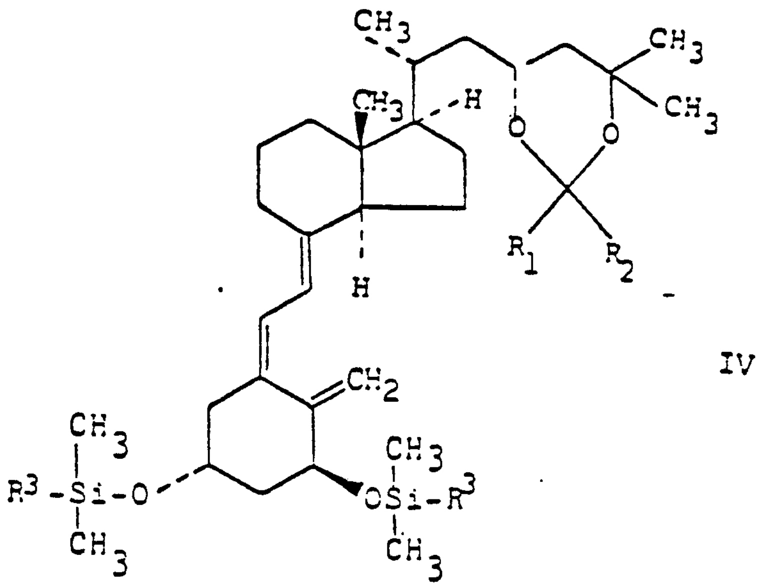

IV

où R1, R2 et R3 ont la signification donnée ci-dessus, et

b) on sépare les groupes protecteurs du composé de formule IV.

2. Composé de formule

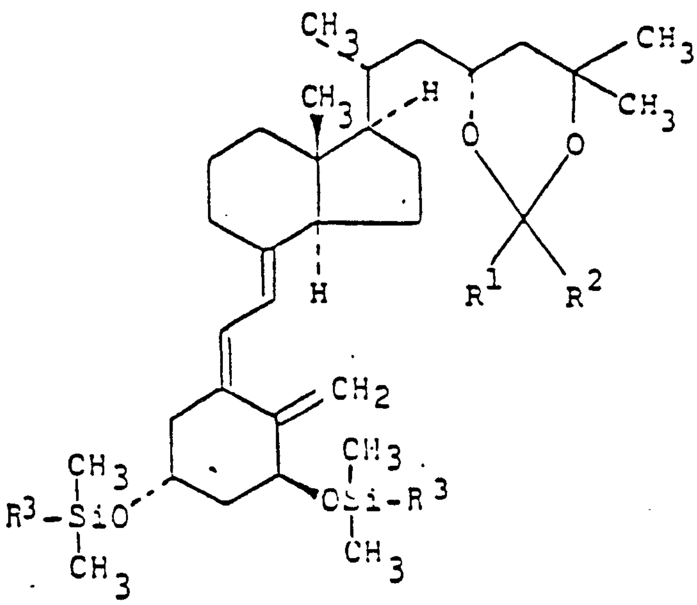

IV

où R1 et R2 représentent un alcoyle inférieur, aryle ou hydrogène avec la configuration R en position 23.

3. Acétal cyclique du (1α,3β,5Z,7E,23R)-9,10-secocholesta-5,7,10(9)-triène-1,3-bis/(1,1-diméthyléthyl)-diméthyl-silyloxy/-23,25-diol-(1-méthyléthylidène).

4. Le 1α,23(R),25-trihydroxycholécalciférol.

**Revendication** pour l'Etat contractant AT

Procédé de préparation du 1α,23(R),25-trihydroxy-cholécalciférol de formule

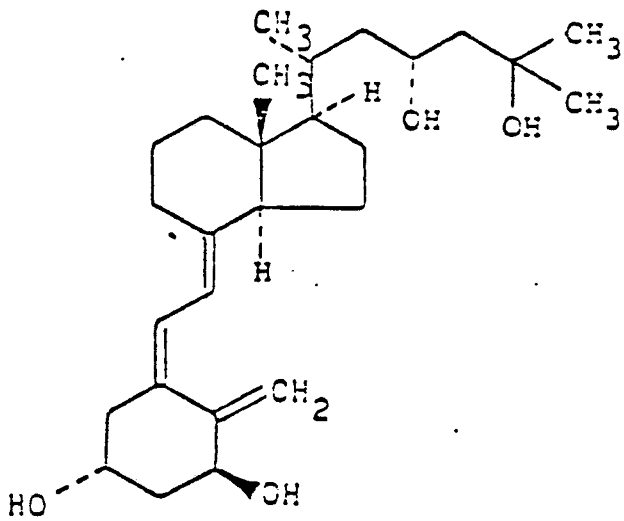

I

caractérisé en ce que
a) on fait réagir un composé de formule

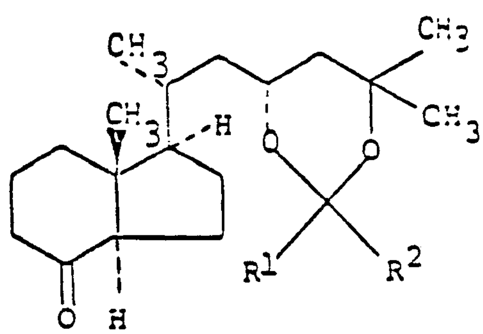

II

où R1 et R2 sont hydrogène, alcoyle inférieur ou aryle, ou forment ensemble un alcoylène en C3 à C6, avec un composé de formule

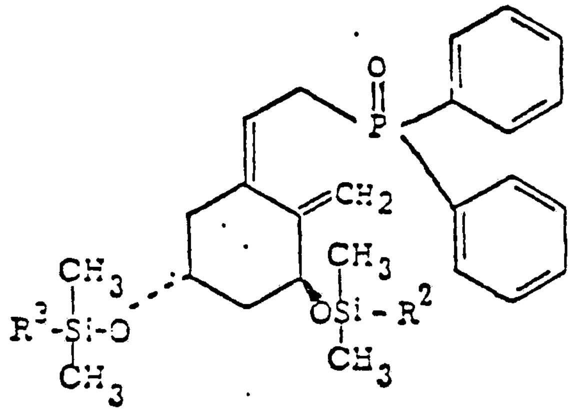

III

où R3 représente un alcoyle inférieur ou un aryle, pour donner un composé de formule

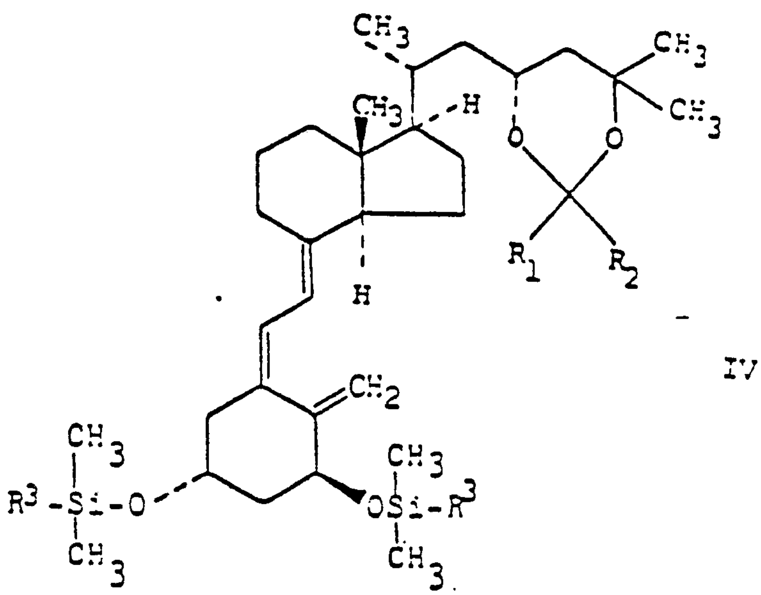

IV

où R1, R2 et R3 ont la signification donnée ci-dessus et

b) on sépare les groupes protecteurs du composé de formule IV.